# EUROPEAN PATENT SPECIFICATION

(11) **EP 1 185 292 B1**
(45) Date of publication and mention of the grant of the patent: **04.05.2005**
(21) Application number: 00938215.1
(22) Date of filing: 08.06.2000
(51) Int. Cl.: A61K 38/20, A61P 7/00, A61P 9/10, A61P 1/00

(54) **USE OF INTERLEUKIN-11 TO TREAT HEMORRHAGIC SHOCK**
VERWENDUNG VON INTERLEUKIN-11 ZUR BEHANDLUNG VON HÄMORAGISCHEM SCHOCK
UTILISATION D'INTERLEUKINE-11 DANS LE TRAITEMENT DU CHOC HEMORRAGIQUE

(30) Priority: 08.06.1999 US 138054 P
(43) Date of publication of application: 13.03.2002
(73) Proprietor: Genetics Institute, LLC, Cambridge, MA 02140 (US)
(72) Inventor: POLLACK, Matthew, Bethesda, MD 20817 (US); KEITH, James, C., Jr., Andover, MA 01810 (US)
(74) Representative: Taylor, Kathryn May
(86) International application number: PCT/US2000/015737
(87) International publication number: WO 2000/074707

(56) References cited:
- US-A- 5 460 810
- FAIST ET AL.: "Therapeutic immunomodulatory approaches for the control of systemis inflammatory response syndrome and the prevention of spesis" NEW HORIZONS, vol. 6, no. 2, 1998, pages s97-s102, XP000971422
- DU ET AL.: "Protective effects of interleukin 11 in a murine model of ischemic bowel necrosis" AM. J. PHYSIOL., vol. 272, no. 3, 1997, pages G545-G552, XP000971485
- MISRA ET AL.: "Recombinanat human interleukin 11 prevents hypotension in LPS-treated anesthetised rabbits" J. ENDOTOXIN RES., vol. 3, no. 4, 1996, pages 297-305, XP000971496
- WANG ET AL.: "Effects of recombinant human endothelial-derived interleukin 8 on hemorrhagic shock in rats" ACTA PATHOLOGICA SINICA, vol. 18, no. 5, 1997, pages 434-436, XP000971506

## Description

### RELATED APPLICATIONS

This application claims the benefit of U.S. Provisional Application No. 60/138,054 filed June 8, 1999.

### FIELD OF INVENTION

The present invention relates to the field of prevention and treatment of hemorrhagic shock using interleukin-11.

### BACKGROUND OF THE INVENTION

Hemorrhagic shock ensues whenever acute blood loss exceeds approximately 20% of normal blood volume. The central feature of hemorrhagic shock is the reduced circulating blood volume which results in hypoperfusion of major organ systems. The hypoperfusion causes local tissue ischemia and nutrient substrate deficiencies that lead to progressive multi-organ damage and subsequent dysfunction (Keith, JC, "Effect of lidocaine pretreatment on acute hemorrhagic shock in the rat," *Circulatory Shock* **19**:283-292 (1986). When resuscitation by intravascular volume replacement with blood or physiologic fluid solutions is initiated, further damage mediated by reperfusion injury occurs (Keith, *supra*).

The gastrointestinal system is severely damaged during hemorrhagic shock and resuscitation. Necrosis of the bowel occurs, and subsequent bacterial translocation with endotoxin release are commonly observed (Tamion, F., et al., *Am. J. Physiol*. **273**(2 Pt 1):G314-21 (1997)). This often results in marked up-regulation of proinflammatory cytokines which can worsen the multiple organ disfunction.

Therefore an agent such as recombinant human interleukin-11("rhIL-11") that has protective effects on the gastrointestinal system and decreases proinflammatory cytokine secretion may be beneficial in the treatment of hemorrhagic shock.

### SUMMARY OF THE INVENTION

Applicants have for the first time determined that interleukin-11 ("IL-11") improves survival of hemorrhagic shock. Thus, provided by the present invention is the use of EL-11 in the manufacture of a medicament for treating and preventing acute hemorrhagic shock.

IL-11, analogs, and derivatives thereof, can be administered to patients, either prophylactically or after the onset of symptoms associated with the aforementioned disorder. IL-11 can be administered in suitable pharmaceutically acceptable carriers either alone or in combination with other conventional agents useful in alleviating the symptoms associated with these disorders.

A method of preventing acute hemorrhagic shock comprises administering to a mammal, prior to the on-set of symptoms, a therapeutically effective amount of interleukin-11.

A method of treating acute hemorrhagic shock comprises administering to a mammal experiencing this disorder a therapeutically effective amount of interleukin-11.

In preferred embodiments, the therapeutic dose is effective to prevent or treat hemorrhagic shock. Preferably, the therapeutically effective amount of interleukin-11 comprises between about 0.1 µg/kg and about 100 mg/kg body weight, more preferably between about 1 µg/kg and about 10 mg/kg body weight, and most preferably between about 10 µg/kg and about 1 mg/kg body weight.

### DETAILED DESCRIPTION OF THE INVENTION

Provided by the present invention is the use of IL-11 in the manufacture of a medicament for treating and preventing acute hemorrhagic shock.

IL-11 is a multifunctional cytokine derived from a stromal cell line (Paul, S.R., et al., *Proc. Natl. Acad. Sci. USA* (1990) **87**:7512-7512) which can stimulate megakaryocyte and platelet production (Bruno, E., et al., *Exp. Hematol*. (1991) **19**:378-385), regulate proliferation and differentiation of macrophage precursors (Du, X.X. and D.A. Williams, *Blood* (1994) **83**:2023-2030), and stimulate B cell immunoglobulin production via a T lymphocyte-dependent mechanism (Yin, T., et al., *J. Exp. Med*. (1992) **175**:211-215). Recent studies have shown prophylactic protective effects of IL-11 on intestinal mucosal damage due to radiation and chemotherapy (Du, X.X., et al., *Blood* (1994) **83**:33-37) and ischemic bowel necrosis (Du, X.X., et al., *Am. J. Physiol*. (1997) **272**:G545-G552). Further, the degree of radiation-induced thoracic injury (Redlich, C.A., et al., *J*. *Immunol*. (1996) **157**:1705-1710) and mortality due to endotoxemia are also reduced by administration of IL-11, indicating that IL-11 may act as an anti-inflammatory cytokine. (Barton, B.E., et al., *Infect. Immun*. (1996) **64**:714-718; Castagliuolo, I., et al., *Am. J. Physiol*. (1997) **273**:G333-G341; and Misra, B.R., et al., *J. Endotoxin Res.* (1996) **3**:297-305)

IL-11 is described in detail in International Application PCT/US90/06803, published May 30, 1991; as well as in U.S. Patent No. 5,215,895; issued June 1, 1993. A cloned human IL-11 was previously deposited with the ATCC, 10801 University Boulevard, Manassa, VA 20110-2209, on March 30, 1990 under ATCC No. 68284. Moreover, as described in U.S. Patent No. 5,270,181; issued December 14, 1993; and U.S. Patent No. 5,292,646; issued March 8, 1994; IL-11 may also be produced recombinantly as a fusion protein with another protein. IL-11 can be produced in a variety of host cells by resort to now conventional genetic engineering techniques. In addition, IL-11 can be obtained from various cell lines, for example, the human lung fibroblast cell line, MRC-5 (ATCC Accession No. CCL 171) and Paul *et al*., the human trophoblastic cell line, TPA30-1 (ATCC Accession No. CRL 1583). Described in *Proc. Natl. Acad. Sci. USA* **87**:7512 (1990) is a cDNA encoding human IL-11 as well as the deduced amino acid sequence (amino acids 1 to 199). U.S. Patent No. 5,292,646, *supra,* describes a des-Pro form of IL-11. in which the N-terminal proline of the mature form of IL-11 (amino acids 22-199) has been removed (amino acids 23-199). As is appreciated by one skilled in the art, any form of IL-11, which retains IL-11 activity, is useful according to the present invention.

In addition to recombinant techniques, IL-11 may also be produced by known conventional chemical synthesis. Methods for constructing the polypeptides useful in the present invention by synthetic means are known to those of skill in the art. The synthetically constructed cytokine polypeptide sequences, by virtue of sharing primary, secondary, or tertiary structural and conformational characteristics with the natural cytokine polypeptides are anticipated to possess biological activities in common therewith. Such synthetically constructed cytokine polypeptide sequences or fragments thereof, which duplicate or partially duplicate the functionality thereof may also be used in the method of this invention. Thus, they may be employed as biologically active or immunological substitutes for the natural, purified cytokines useful in the present invention.

Modifications in the protein, peptide or DNA sequences of these cytokines or active fragments thereof may also produce proteins which may be employed in the methods of this invention. Such modified cytokines can be made by one skilled in the art using known techniques. Modifications of interest in the cytokine sequences, *e.g*., the IL-11 sequence, may include the replacement, insertion or deletion of one or more selected amino acid residues in the coding sequences. Mutagenic techniques for such replacement, insertion or deletion are well known to one skilled in the art. (See, *e.g*., U. S. Patent No. 4,518,584.)

Other specific mutations of the sequences of the cytokine polypeptides which may be useful therapeutically as described herein may involve, *e*.*g*., the insertion of one or more glycosylation sites. An asparagine-linked glycosylation recognition site can be inserted into the sequence by the deletion, substitution or addition of amino acids into the peptide sequence or nucleotides into the DNA sequence. Such changes may be made at any site of the molecule that is modified by addition of O-linked carbohydrate. Expression of such altered nucleotide or peptide sequences produces variants which may be glycosylated at those sites.

Additional analogs and derivatives of the sequence of the selected cytokine which would be expected to retain or prolong its activity in whole or in part, and which are expected to be useful in the present method, may also be easily made by one of skill in the art. One such modification may be the attachment of polyethylene glycol (PEG) onto existing lysine residues in the cytokine sequence or the insertion of one or more lysine residues or other amino acid residues that can react with PEG or PEG derivatives into the sequence by conventional techniques to enable the attachment of PEG moieties.

Additional analogs of these selected cytokines may also be characterized by allelic variations in the DNA sequences encoding them, or induced variations in the DNA sequences encoding them. It is anticipated that all analogs disclosed in the above-referenced publications, including those characterized by DNA sequences capable of hybridizing to the disclosed cytokine sequences under stringent hybridization conditions or non-stringent conditions (Sambrook *et al*., Molecular Cloning. *A Laboratory Manual*, 2d edit., Cold Spring Harbor Laboratory, New York (1989)) will be similarly useful in this invention.

Also considered useful in these methods are fusion molecules, prepared by fusing the sequence or a biologically active fragment of the sequence of one cytokine to another cytokine or proteinaceous therapeutic agent, *e.g.,* IL-11 fused to IL-6 (see, *e*.*g*., methods for fusion described in PCT/US91/06186 (WO92/04455), published March 19, 1992). Alternatively, combinations of the cytokines may be administered together according to the method.

Thus, where in the description of this invention IL-11 is mentioned by name, it is understood by those of skill in the art that IL-11 encompasses the protein produced by the sequences presently disclosed in the art, as well as proteins characterized by the modifications described above yet which retain substantially similar activity.

Pharmaceutical compositions containing IL-11 which are useful in the present invention may also contain pharmaceutically acceptable carriers, diluents, fillers, salts, buffers, stabilizers and/or other materials well-known in the art. The term "pharmaceutically acceptable" means a material that does not interfere with the effectiveness of the biological activity of the active ingredient (s) and that is not toxic to the host to which it is administered. The characteristics of the carrier or other material will depend on the route of administration.

Administration can be carried out in a variety of conventional ways. Intraperitoneal injection is the preferred method of administration. Intravenous, cutaneous or sub-cutaneous injection may also be employed. For injection, IL-11 will preferably be administered in the form of pyrogen-free, parenterally acceptable aqueous solutions.

The preparation of such parenterally acceptable protein solutions, having due regard to pH, isotonicity, stability and the like, is within the skill of the art.

The amount of IL-11 used for treatment will depend upon the severity of the condition, the route of administration, the reactivity or activity of the active ingredient, and ultimately will be decided by the treatment provider. In practicing the methods of treatment of this invention, a therapeutically effective amount of IL-11 is administered. The term "therapeutically effective amount" means the total amount of each active component of the method or composition that is sufficient to show a meaningful patient benefit (e.g., curing, ameliorating, inhibiting, delaying or preventing onset of, preventing recurrence or relapse of). One common technique to determine a therapeutically effective amount for a given patient is to administer escalating doses periodically until a meaningful patient benefit is observed by the treatment provider. When applied to an individual active ingredient, administered alone, the term refers to that ingredient alone. When applied to a combination, the term refers to combined amounts of the active ingredients that result in the therapeutic effect, whether administered in combination, serially or simultaneously. A therapeutically effective dose of IL-11 in this invention is contemplated to be in the range of about 0.1 µg/kg to about 100 mg/kg body weight, more preferably between about 1 µg/kg and about 10 mg/kg body weight, and most preferably between about 10 µg/kg and about 1 mg/kg body weight. The number of administrations may vary, depending on the individual patient and the severity of the disease.

The present inventors have shown, for the first time, the protective role of IL-11 in hemorrhagic shock. IL-11 improves survival in hemorrhagic shock by down-regulating inflammation, preventing tissue injury, facilitating tissue repair, and minimizing shock-related bacterial/endotoxin translocation from the gut.

The present invention is further exemplified and supported by reference to the experimental results described below.

### EXAMPLES

In the following Examples, those marked ∗ are understood to full outside the scope of the claim, but are useful background to assist in the understanding of the invention.

### ∗ EXAMPLE 1: Models of Short Bowel and Intestinal Ischemia / Reperfusion

Interleukin-11 produces intestinal mucosal trophic effects in rats with experimental short bowel syndrome (Liu, Q., et al., *J. Pediatr. Surg.* (1996) 31(8):1047-1050). After 90% small bowel resection, groups of animals were treated with rhIL-11 (125 micrograms/kg twice daily, subcutaneously) or 0.1% bovine serum albumin. The animals were weighed daily and were killed on day 2, 4, 6, or 8; the remaining small intestine was evaluated for villus height and crypt cell mitosis. The body weight of the animals that received IL-11 was significantly greater at the beginning of postoperative day 4 in comparison to that of the bovine serum albumin ("BSA") groups. The rats that had IL-11 also had significantly greater villus height and crypt cell mitotic rates.

The effects of interleukin-11 and epidermal growth factor on residual small intestine were compared after massive small bowel resection in rats (Fiore, N.F., et al., *J. Pediatr. Surg.* (1998) **33**(1):24-29). Body weight was similar at day 4 and day 8 after resection. Animals treated with rhIL-11 and IL-11-EGF had increased mucosal mass at day 4 and 8 when compared with controls and EGF. Muscle thickness was significantly increased in the EGF group. IL-11 had a trophic effect on small intestinal enterocytes, causing cell proliferation and increased mucosal thickness. EGF has a more generalized effect on intestine causing proliferation of both enterocytes and myocytes. IL-11, with or without EGF, may have clinical utility in instances of short bowel syndrome.

Protective effects of interleukin-11 have been shown in a murine model of ischemic bowel necrosis (Du., X., et al., *Am. J. Physiol.* (1997) **272**(3 Pt 1):G545-G552). Pretreatment with rhIL-11 in mice with bowel ischemia (induced by occluding the superior mesenteric artery for 90 min) significantly decreased morbidity and mortality. rhIL-11-treated mice demonstrated rapid recovery of intestinal mucosa, a concurrent increase in mitotic activity, a suppression of apoptosis in intestinal crypt cells, and an increased peripheral platelet and leukocyte count. Vehicle-treated mice developed thrombocytopenia after ischemia, but no rhIL-11-treated mice developed thrombocytopenia.

### ∗ EXAMPLE 2: Models of Systemic Inflammatory Conditions

rhIL-11 improves survival in the neutropenic rat model of *Pseudomonas* sepsis (Opal, S.M., et al., *J. Infect. Dis*. (1998) **178**:1205-1208). rhIL-11 (150 µg/kg) was administered intravenously ("IV") once daily for three days, beginning at the onset of fever in cyclophosphamide treated rats that were colonized in the gastrointestinal tract with *Pseudomonas aeruginosa* 12.4.4 at the beginning of the experiment. rhIL-11-treated animals had a significant reduction in the quantitative level of lung infection; reduction in pulmonary edema and marked reduction in injury to the gastrointestinal epithelium of the small and large intestine. Forty percent of rhIL-11, 60% of ciprofloxacin and 100% of rhIL-11-ciprofloxacin treated animals survived. These results indicate that rhIL-11 supports mucous membrane integrity of the alimentary tract and decreases the systemic inflammatory response to experimental gram-negative infection in immunocompromised animals.

In BALB/c mice given doses of D-galactosamine and *Staphylococcus aureus* enterotoxin B as a model of T-cell mediated toxic shock triggered by superantigen (Barton, B.E., et al., *Infect. Immun*. (1996) **64**:714-718), rhIL-11 (5 to 500 µg/kg IP) given as a pretreatment 1 hour before superantigen decreased mortality in a dose-dependent fashion at 48 hours. At 500 µg/kg, mortality was 55% compared to 100% in BSA-treated animals.

rhIL-11 has been evaluated in a rabbit model of endotoxemia (Misra, B.R., et al., *J. Endotoxin Res*. (1996)**3**:297-305). Lipopolysaccharide(LPS) was administered IV and 30 minutes later vehicle or rhIL-11 (100 µg/kg IV) was administered. Mean arterial pressure of the vehicle treated group was 55% of baseline 5 hours after LPS administration, while that of rhIL-11 treated animals was 94% of baseline. Histologic evaluation of the ileum, cecum, and colon showed decreased hemorrhage, edema, and mucosal damage in rhIL-11 treated animals compared to vehicle treated animals. rhIL-11 treated animals displayed significantly lower plasma nitrate/nitrite levels compared to vehicle treated animals. These results demonstrate that rhIL-11 can prevent hypotension that occurs as a consequence of endotoxemia, and that this effect is associated with decreased plasma nitric oxide levels.

In a murine model of endotoxemia the effect of rhIL-11 on serum levels of proinflammatory cytokines was evaluated (Trepicchio, W.L., et al., *J. Immunol*. (1996) **157**:3627-3634). Female C57BL/6J mice were injected with PBS or rhIL-11 (500 µg/kg IP) and 4 hours later injected with PBS or LPS. Animals treated with rhIL-11 prior to LPS administration had significantly lower peak TNF-α, IFN-γ, and IL-1β serum levels, ranging from 80 to 95% reductions, compared to animals which received only LPS. rhIL-11 pretreatment did not effect LPS-induced IL-6 or IL-10 production and rhIL-11 treatment alone did not result in detectable serum levels of IL-6 or IL-10. rhIL-11 also inhibited LPS-induced TNF-α production in the IL-6 deficient knockout mouse. Therefore, rhIL-11 can reduce proinflammatory cytokine production during a systemic inflammatory response in vivo and this activity is not dependent on induction of IL-10 or IL-6.

Interleukin-11 improved survival and reduces bacterial translocation and bone marrow suppression in burned mice (Schindel, D., et al., *J. Pediatr. Surg.* (1997) **32**(2):312-315). The effect of IL-11 on survival, intestinal cytoarchitecture, bacterial translocation, and bone marrow suppression in a highly lethal murine burn model was assessed. C3H/HeJ, 8 to 10-week-old mice, underwent a standardized 32% total body surface area (TBSA) scald burn using a burn template. Mice were divided equally between groups receiving IL-11 (125 micrograms/kg, twice daily, subcutaneously ("SC")) and control (BSA). Survival was calculated to 7-days postburn. At 24-hours postburn, IL-11-treated mice had less enteric bacteria in mesenteric lymph, increased intestinal crypt cell and intestinal villus height, increased peripheral platelet and lymphocyte counts, and an improved survival compared with controls. These data demonstrate that IL-11 improved survival, intestinal cytoarchitecture, reduced bacterial translocation, and reduced bone marrow suppression after a 32% TBSA burn in mice.

### EXAMPLE 3: Hemorrhagic Shock

Sprague-Dawley rats were anesthetized and bled 28 ml/kg (35% blood volume) over 10 minutes to induce acute hemorrhagic shock. After 75 minutes of shock, the animals were randomized to different treatment groups. They were then resuscitated for 60 minutes with 3:1 (fluid : blood loss) lactated Ringer's solution IV containing rhIL-11 (100 µg/kg), anti-IL-11 (5.7 mg/kg) or only Lactated Ringer's solution. Blood pressures were measured to 3 hours after shock. Parallel groups of animals were then either euthanized at 3 hours or were allowed to awaken from anesthesia and observed to 72 hours after shock. The 72-hour mortality rate was determined, and then the animals were euthanized. At necropsy, specimens of ileum were fixed in 10% buffered neutral formalin and subsequently examined to evaluate the degree of small intestinal damage. Additional specimens of liver and ileum were obtained for quantification of cytokine mRNA levels by RT-PCR. The sections of ileum were examined microscopically by an evaluator without knowledge of treatment group. The intestinal specimens were scored for the degree of: Villus atrophy; Crypt atrophy; Goblet cell depletion; Segment Dilation; and Inflammatory cell infiltrate. A score of 0 indicated no lesion while a score of 3 indicated severe damage and necrosis.

Ninety-two percent of the rhIL-11 animals survived until 72 hours while only 72% and 79% of the lactated Ringer's solution alone and lactated Ringer's solution-anti-IL-11 group survived, respectively. The levels of Tumor necrosis factor alpha (TNF-α) expression are seen below. In both the liver and ileum, treatment with rhIL-11 at the time of resuscitation significantly reduced TNF-α expression.

**Table 1.**

| RT-PCR TNF-a specific mRNA levels at 72 hours | | |
|---|---|---|
| Group | Ileum | Liver |
| LR | 3.8"0.17 | 3.7"0.17 |
| LR-Anti-IL-11 | 4.0"1.0 | 4.9"0.75 |
| LR-rhIL-11 | 1.8"0.17* | 1.6"0.18H |

| | | |
|---|---|---|
| *P=0.0089 HP=0.0002 | | |

The histologic results are shown in Table 2. rhIL-11 added to the resuscitation fluid reduced the histologic lesion scores at 3 and 72 hours. The differences were highly significant at 72 hours. Representative photomicrographs from 72-hour specimens are seen in Figure 1. Of special note is the complete destruction of the villi in the lactated Ringer's solution group, while the villi appear relatively normal in the rhIL-11 group.

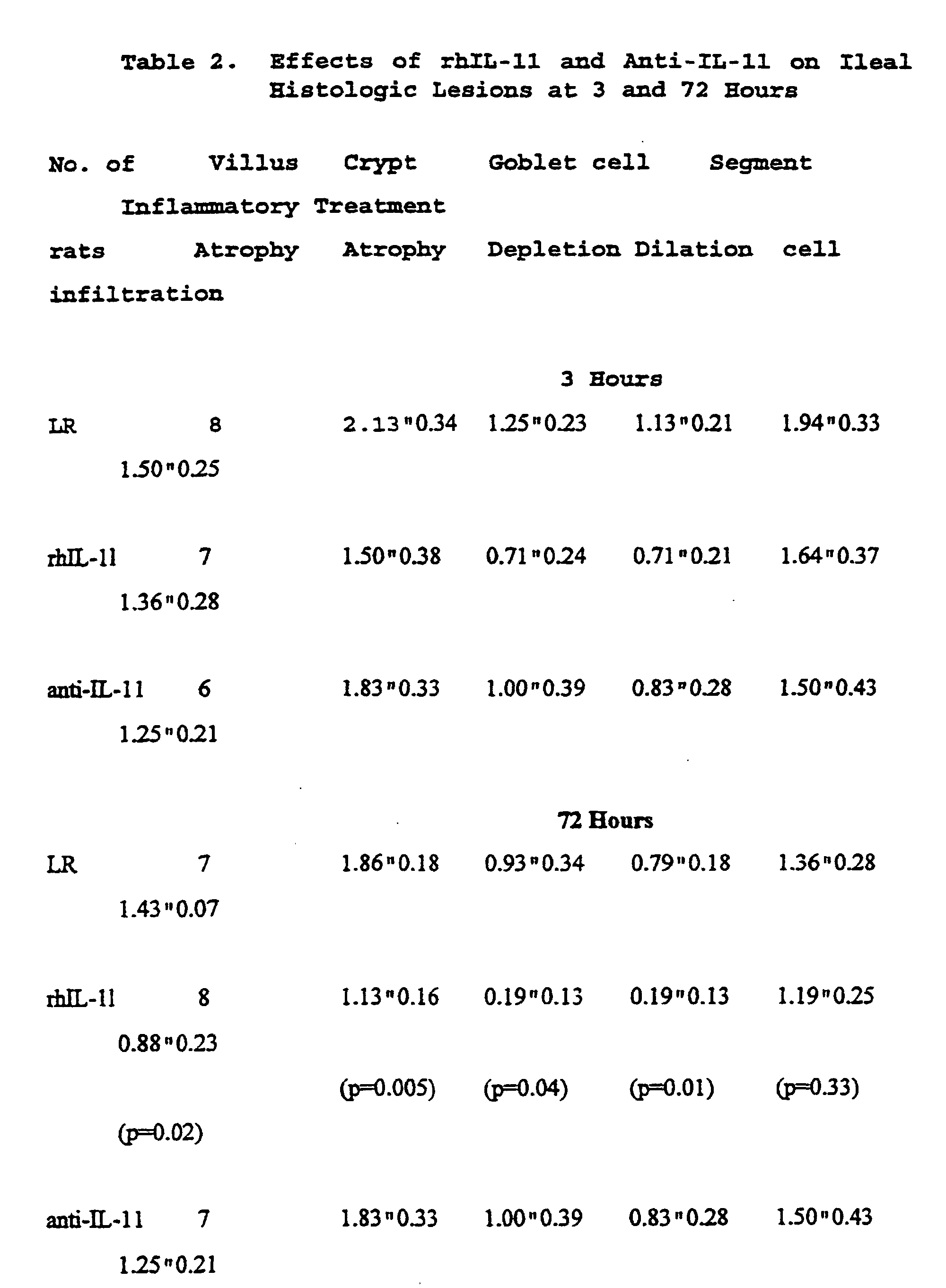

As shown in Table 2 above, rhIL-11 added to the resuscitation fluid suppressed the expression of TNF-α in the liver and ileum, decreased the intestinal damage at 72 hours and improved the survival following hemorrhagic shock.

Additionally, in experiments substantially similar to that described above in Example 3, expression of TNF, IL-6, proinflammatory cytokines and VCAM-1, ICAM-1, adhesion moleucles,is reduced in liver and ileum cells from rats resuscitated with rhIL-11 containing lactated Ringer's solution compared to expression of the same molecules in the liver and ileum cells from control rats resuscitated with only lactated Ringer's solution. While the expression level of IL-10,an anti-inflammatory cytokine, from liver and ileum cells of rhIL-11 resuscitated rats appears comparable to that of the same cells from the untreated control rats. Furthermore, the mean arterial pressure of rhIL-11 resuscitated rats is higher than that of the untreated control rats.

In conclusion, IL-11 improves survival in hemorrhagic shock by downregulating inflammation, preventing tissue injury, facilitating tissue repair, and minimizing shock-related bacterial/endotoxin translocation from the gut.

### REFERENCES:

Keith JC: Effect of lidocaine pretreatment on acute hemorrhagic shock in the rat. Circulatory Shock 19:283-292, 1986.
Tamion F, Richard V, Lyoumi S, Daveau M, Bonmarchand G, Leroy J, Thuillez C, Lebreton JP. Gut ischemia and mesenteric synthesis of inflammatory cytokines after hemorrhagic orendotoxic shock. Am J Physiol 1997; 273(2 Pt 1):G314-21.
Liu Q, Du XX, Schindel DT, Yang ZX, Rescorla FJ, Williams DA, Grosfeld JL. Trophic effects of interleukin-11 in rats with experimental short bowel syndrome. J Pediatr Surg 1996 Aug;31(8):1047-50; discussion 1050-1.
Fiore NF, Ledniczky G, Liu Q, Orazi A, Du X, Williams DA, Grosfeld JL. Comparison of interleukin-11 and epidermal growth factor on residual small intestine after massive small bowel resection. J Pediatr Surg 1998 Jan;33(1):24-9.
Du X, Liu Q, Yang Z, Orazi A, Rescorla FJ, Grosfeld JL, Williams DA. Protective effects of interleuldn-11 in a murine model of ischemic bowel necrosis. Am J Physiol 1997 Mar;272(3 Pt 1):G545-52.
Opal SM, Jhung JW, Keith Jr., JC, et al. 1998. Recombinant human interleukin-11 in experimental pseudomonas aeruginosa sepsis in immunocompromised animals. J Infect Dis. 178:1205-1208.
Barton BE, Shortall J, Jackson JV. 1996. Interleukins 6 and 11 protect mice from mortality in a staphylococcal enterotoxin-induced toxic shock model. Infect and Immun. 64:714-718.
Misra BR, Ferranti TJ, Keith, Jr JC, et al. 1996. Recombinant human interleukin-11 prevents hypotension in LPS-treated anesthetized rabbits. J Endotoxin Res. 3:297-305.
Trepicchio WL, Bozza M, Pedneault G, Dorner AJ. 1996. Recombinant human interleukin-11 attenuates the inflammatory response through downregulation of proinflammatory cytokine release and nitric oxide production. J Immunol. 157:3627-3634.
Schindel D, Maze R, Liu Q, Williams D, Grosfeld J. Interleukin-11 improves survival and reduces bacterial translocation and bone marrow suppression in burned mice. J Pediatr Surg 1997 Feb;32(2):312-5.

## Claims

1. The use of interleukin-11 in the manufacture of a medicament for treating and preventing acute hemorrhagic shock.

2. The use according to claim 1 wherein the medicament is for delivery of interleukin-11 in an amount of from 1 to 1000 µg/kg body weight.

3. The use of claim 1 or claim 2, wherein said interleukin-11 has the amino acid sequence of a human IL-11 polypeptide.

4. The use of any preceding claim, wherein said interleukin-11 is recombinantly produced.

5. The use of any preceding claim, wherein said medicament is for intraperitoneal administration.

6. The use of any of claims 1 to 4, wherein said medicament is formulated for intravenous, cutaneous, or sub-cutaneous administration.

7. The use of any preceding claim, wherein said medicament is formulated for an effective dose in the range of about 0.1 µg/kg to about 100 mg/kg body weight.

8. The use of any preceding claim, wherein said medicament is formulated for an effective dose in the range of about 1 µg/kg to about 10 mg/kg body weight.

9. The use of any preceding claim, wherein said medicament is formulated for an effective dose in the range of about 10 µg/kg to about 1 mg/kg body weight.

## Patentansprüche

1. Verwendung von Interleukin-11 bei der Herstellung eines Medikaments zur Behandlung oder Vorbeugung des akuten hemorrhagischen Schocks.

2. Verwendung nach Anspruch 1, worin das Medikament für die Übermittlung von Interleukin-11 in einer Menge von 1 bis 1.000 µg/kg Körpergewicht bestimmt ist.

3. Verwendung nach Anspruch 1 oder Anspruch 2, worin das Interleukin-11 die Aminosäuresequenz eines humanen IL-11-Polypeptids hat.

4. Verwendung nach einem der vorstehenden Ansprüche, worin das Interleukin-11 rekombinant erzeugt ist.

5. Verwendung nach einem der vorstehenden Ansprüche, worin das Medikament für die intraperitonale Verabreichung ist.

6. Verwendung nach einem der Ansprüche 1 bis 4, worin das Medikament für die intravenöse, cutane oder subcutane Verabreichung formuliert ist.

7. Verwendung nach einem der vorstehenden Ansprüche, worin das Medikament für eine effektive Dosis im Bereich von etwa 0,1 µg/kg bis etwa 100 mg/kg Körpergewicht formuliert ist.

8. Verwendung nach einem der vorstehenden Ansprüche, worin das Medikament für eine effektive Dosis im Bereich von etwa 1 µg/kg bis etwa 10 mg/kg Körpergewicht formuliert ist.

9. Verwendung nach einem der vorstehenden Ansprüche, worin das Medikament für eine effektive Dosis im Bereich von etwa 10 µg/kg bis etwa 1 mg/kg Körpergewicht formuliert ist.

## Revendications

1. Utilisation d'interleukine-11 dans la fabrication d'un médicament destiné au traitement et à la prévention du choc hémorragique aigu.

2. Utilisation selon la revendication 1, dans laquelle le médicament est destiné à l'administration d'interleukine-11 dans une quantité de 1 à 1000 µg/kg de poids corporel.

3. Utilisation selon la revendication 1 ou la revendication 2, dans laquelle ladite interleukine-11 possède la séquence d'acides aminés d'un polypeptide IL-11 humain.

4. Utilisation selon l'une quelconque des revendications précédentes, dans laquelle ladite interleukine-11 est produite de façon recombinante.

5. Utilisation selon l'une quelconque des revendications précédentes, dans laquelle ledit médicament est destiné à une administration intrapéritonéale.

6. Utilisation selon l'une quelconque des revendications 1 à 4, dans laquelle ledit médicament est formulé pour une administration intraveineuse, cutanée, ou sous-cutanée.

7. Utilisation selon l'une quelconque des revendications précédentes, dans laquelle ledit médicament est formulé pour une dose efficace dans la plage d'environ 0,1 µg/kg jusqu'à environ 100 mg/kg de poids corporel.

8. Utilisation selon l'une quelconque des revendications précédentes, dans laquelle ledit médicament est formulé pour une dose efficace dans la plage d'environ 1 µg/kg jusqu'à environ 10 mg/kg de poids corporel.

9. Utilisation selon l'une quelconque des revendications précédentes, dans lequel ledit médicament est formulé pour une dose efficace dans la plage d'environ 10 µg/kg jusqu'à environ 1 mglkg de poids corporel.
